# EUROPÄISCHE PATENTANMELDUNG

(11) **EP 0 982 048 A1**
(43) Veröffentlichungstag der Anmeldung: **01.03.2000**
(21) Anmeldenummer: 98104492.8
(22) Anmeldetag: 12.03.1998
(51) Int. Cl.: A61M 27/00

(54) **Implantat zur kontrollierten Ableitung von Gehirnflüssigkeit**

(71) Anmelder: Leonhardt, Steffen, Dr.-Ing., 23566 Lübeck (DE); Isermann, Rolf, Prof.-Dr.-Ing. Dr.h.c., 64342 Seeheim (DE); Walter, Marian, Dipl.-Ing., 64287 Darmstadt (DE); Steudel, Wolf Ingo, Prof. Dr. med., 66424 Homburg/Saar (DE)
(72) Erfinder: Leonhardt, Steffen, Dipl.-Ing., 23566 Lübeck (DE); Walter, Marian, Dipl.-Ing., 64287 Darmstadt (DE); Isermann, Rolf, Prof. Dr.-Ing. Dr.h.c., 64342 Seeheim (DE); Steudel, Wolf Ingo, Prof. Dr.med., 66424 Homburg/Saar (DE)
(74) Vertreter: von Fischern, Bernhard, Dipl.-Ing.

(57) **Zusammenfassung**

Die Erfindung betrifft ein Implantat zur kontrollierten Ableitung von Gehirnflüssigkeit, insbesondere zur Therapie des Wasserkopfes, mit einem unter der Haut verlegten Katheter (3), dessen erstes freies Ende (5) zu einer Stelle im Gehirn geführt ist, von wo überschüssige Gehirnflüssigkeit ableitbar ist, und dessen zweites freies Ende zu einer Stelle im Körper geführt ist, von wo die überschüssige Gehirnflüssigkeit vom Körper aufnehmbar ist, und mit einem Ventil, das in dem Katheter auf dessen Verlauf eingebracht ist. Um dem behandelnden Arzt weitere Diagnose- und Therapiemöglichkeiten zu eröffnen, ist eine Telemetrieeinheit (51) vorgesehen, die eine Meßeinheit mit mindestens einem daran angeschlossenen Sensor und eine Stelleinheit mit mindestens einem daran angeschlossenen Aktor aufweist, wobei mindestens ein Zustand des Implantats seitens der Telemetrieeinheit über die Meßeinheit meßbar und über die Stelleinheit verstellbar ist. Zwischen der Telemetrieeinheit und einer außerhalb des Körpers befindlichen Kontrolleinheit ist eine Datenübertragung und/oder eine Energieübertragung herstellbar.

## Beschreibung

Die Erfindung betrifft ein Implantat zur kontrollierten Ableitung von Gehirnflüssigkeit, insbesondere zur Therapie des Wasserkopfes, nach dem Oberbegriff des Patentanspruchs 1.

Unter einem Wasserkopf (lateinisch Hydrocephalus) versteht man die krankhaft erhöhte Ansammlung von Gehirnflüssigkeit im Inneren des Schädels. Dabei ist das empfindliche Gleichgewicht von Gehirnflüssigkeitsproduktion und deren Aufnahme gestört, so daß die Gehirnflüssigkeit permanent, intermittierend oder gelegentlich unter krankhaft erhöhtem Druck steht.

Es ist bekannt, zur Therapie des Wasserkopfes ein Drainagesystem, das als "Shunt" bezeichnet wird, zu implantieren. Ziel ist die Normalisierung des Drucks durch Schaffung eines druckgesteuerten Abflusses. Der Shunt bildet dabei eine Einheit aus einem Katheter und einem Ventil. Das erste freie Ende des Katheters wird durch den Schädel und die harte Hirnhaut ins Innere des Gehirns geführt. Während der Katheter hierzu auf seinem ersten freien Ende aus einem versteiften Material besteht, geht der Katheter an dem oberen Rand des Schädels in einen Silikonschlauch über, der flexibel im Körper verlegt werden kann. Auf seinem Verlauf ist ein Ventil eingebracht, das einen druckgesteuerten Abfluß der Gehirnflüssigkeit bewirkt.

Die bisher verwendeten Ventile stellen eine Art Sicherheitsventil mit Rückflußsperre dar. So sind beispielsweise Ventile bekannt, bei denen eine Kugel durch eine Feder in einen Konus gedrückt wird. Liegt ein bestimmter Druck der Gehirnflüssigkeit am Eingang des Ventils an, so wird die Kugel entgegen der Federkraft aus dem Konus gedrückt, so daß Flüssigkeit durch das Ventil strömen kann. Bei bestimmten Ventilen ist zusätzlich eine mechanische Verstellmöglichkeit von außen vorgesehen, bei der der Öffnungsdruck, bei dem sich das Ventil zur Ableitung der Gehirnflüssigkeit öffnet, in groben Bereichen verstellbar ist. In umgekehrter Flußrichtung wird durch den Ventilsitz der Kugel in dem Konus ein Rückfluß verhindert. Eine Verhinderung des Rückflusses ist schon deshalb notwendig, da bereits ein Tropfen Blut ein derart mechanisch konstruiertes Ventil komplett verkleben und somit das Ventil funktionsunfähig machen könnte. Es ist deshalb auch bekannt, vor dem Ventil eine Erweiterung des Silikonschlauchs (Pumpkammer) und ein zusätzliches Rückschlagventil vorzusehen. Durch pumpenartiges Drücken auf die Erweiterung können dann eventuelle Verunreinigungen noch beseitigt werden.

Die Positionierung des Ventils im Körper ist im Prinzip beliebig. Es hat sich allerdings als vorteilhaft erwiesen, die Ventile unter der Haut hinter dem Ohr einzusetzen. Das von dem Ventil abgehende zweite freie Ende des Katheters wird von dort unter der Haut in den Bauchraum geführt, wo die überschüssige Hirnflüssigkeit vom Körper aufgenommen werden kann.

Seit nahezu 40 Jahren werden derartige Shunt-Systeme zur Therapie des Wasserkopfes implantiert. Dennoch arbeiten bekannte Shunt-Systeme nicht zuverlässig, sondern weisen im Alltagsbetrieb eine Überdrainage oder eine Unterdrainage auf. Überdrainage bewirkt ein Leerlaufen des Gehirninneren, das spontane Entlastungsblutungen und Kopfschmerzen zur Folge hat. Zudem kann eine Überdrainage zu einem Zusammenfallen des Gehirninneren führen und damit Schädigungen des zentralen Nerven-systems hervorrufen (Schlitzventrikelsyndrom). Eine Unterdrainage ist für den Patienten ebenfalls gefährlich, da durch den steigenden Druck Hirnsubstanz zerstört werden kann. Allerdings ist durch eine mäßige Unterdrainage auch eine Therapie möglich, da dann die natürlichen Vorgänge zur Absorption der Gehirnflüssigkeit wieder angeregt werden können.

Das Problem der Über- bzw. Unterdrainage wird zum einen durch die bestehenden Ventilkonstruktionen selber hervorgerufen. So weisen auch Ventile gleicher Baureihe eine Streuung des Öffnungsdruckes auf. Zum anderen ist aber auch der optimal einzustellende Öffnungsdruck individuell von dem jeweiligen Patienten abhängig und kann vor der Implantation des Shunt-Systems nicht bestimmt werden. Ein weiteres Problem ist die Anpassung des hydraulischen Verhaltens des Implantats an unterschiedliche Körperpositionen. So sinkt beim Aufrichten des Körpers zwar der Druck im Gehirn, es wird aber durch die Höhendifferenz zwischen Eingang und Ausgang des zum Bauch führenden Katheters ein zusätzliches Druckgefälle geschaffen. Auch bei diesem Druckwert sollte das Ventil jedoch nur die gerade notwendige Flüssigkeitsmenge abführen.

Insgesamt hat der behandelnde Arzt nach der Implantation somit nur sehr eingeschränkte Möglichkeiten der Diagnose und Therapie. Eine nicht optimale Anpassung des Ventils an den Patienten oder eine Fehlfunktion kann nur an symptomatischen Beschwerden, z.B. Kopfschmerzen, oder in einem fortgeschrittenen Zustand durch ein Computertomogramm erkannt werden. Selbst bei Ventilen mit mechanischer Verstellmöglichkeit des Öffnungsdrucks stehen dem Arzt somit nur sehr ungenaue Angaben zur Verfügung, wie er den neuen Öffnungsdruck einzustellen hat. Als letzte Therapiemöglichkeit bleibt in den meisten Fällen nur eine erneute Operation, die immer mit großen Belastungen und Risiken für den Patienten und hohen Kosten für die Allgemeinheit verbunden sind.

Aufgabe der vorliegenden Erfindung ist es deshalb, ein Implantat der gattungsgemäßen Art zu schaffen, das weitere Diagnose- und Therapiemöglichkeiten dem behandelnden Arzt auch nach der Implantation ermöglicht.

Diese Aufgabe wird durch die im Patentanspruch 1 aufgeführten Merkmale gelöst. Die erfindungsgemäße Lösung besteht darin, daß die Ableitungs-charakteristik des Implantats von außen mittels einer Telemetrieeinheit meßbar und/oder veränderbar ist. In einem ersten Schritt ermöglicht es die Erfindung, in der Telemetrieeinheit eine Meßeinheit vorzusehen, an die mindestens ein Sensor zur Messung mindestens eines Zustands des Implantats angeschlossen ist. So können z.B. der Druck am ersten freien Ende des Katheters und/oder der Fluß durch den Katheter gemessen und über die Telemetrieeinheit aus dem Körperinneren zu einer Kontrolleinheit übertragen werden. Alternativ oder zusätzlich können mit der erfindungsgemäßen Telemetrieeinheit auch Stellwerte von der Kontrolleinheit zur Telemetrieeinheit übertragen werden, die dann an die angeschlossenen Aktoren weitergeleitet werden. Insbesondere ist es somit möglich, das Ventil als Stellventil auszuführen und somit den Fluß durch den Katheter von außen zu verändern. Weiterhin ermöglicht es die Erfindung, daß in der Telemetrieeinheit ein Regler vorgesehen ist, der das Stellventil selbständig in Abhängigkeit von den erfaßten Meßwerten und einem Sollwert verstellt. Der Sollwert kann wiederum über die Kontrolleinheit vorgegeben und verändert werden.

Das erfindungsgemäße Implantat weist gegenüber herkömmlichen Implantaten eine Reihe von Vorteilen auf. So ist es möglich, das Verhalten des Implantats auch unter Alltagsbedingungen zu erfassen und in regelmäßigen Abständen zu kontrollieren. Falls Abweichungen in dem Systemverhalten festgestellt werden, können die Ventileigenschaften in der gewünschten Weise verändert werden. Dies kann zum einen ebenfalls über die Telemetrieeinheit durch entsprechend vorgesehene Aktoren geschehen, oder aber es sind entsprechende Verstellmöglichkeiten am Ventil vorgesehen, die durch mechanische Einwirkungen von außen auf das Ventil verändert werden können. Falls ein entsprechend fein verstellbarer Aktor im Ventil vorhanden ist, könnte durch eine gezielte Therapie die Resorptionsfähigkeit des Patienten reaktiviert werden. Dies ist durch eine leichte Unterdrainage möglich, die sich auf die patientenspezifischen Bedürfnisse erfindungsgemäß einstellen läßt. So kann im Idealfall das Implantat im Laufe der Jahre überflüssig werden.

Ein weiterer Vorteil der Erfindung besteht darin, daß zwischen der Telemetrieeinheit und der Kontrolleinheit neben der Datenübertragung auch eine Energieübertragung möglich ist. Hierdurch kann ein Akkumulator aufgeladen werden, der parallel zu einer im Implantat vorhandenen Batterie oder auch alleine zur Energieversorgung der im Implantat vorhandenen elektrischen und elektronischen Bauteile dient.

Nach einer bevorzugten Ausführungsform ist das Ventil in der Telemetrieeinheit integriert. Insbesondere kann vorgesehen sein, daß das Ventil und die Telemetrieeinheit eine integrierte Einheit bilden, an die das erste freie Ende und das zweite freie Ende des Katheters anschließbar ist und die unter der Haut implantierbar ist. Als bevorzugte Stelle zur Implantation wird eine Stelle hinter dem Ohr oder unter dem Schlüsselbein gesehen.

An die Meßeinheit sind zweckmäßigerweise ein oder mehrere Drucksensoren angeschlossen, die den Druckabfall an ein oder mehreren Stellen des Katheters messen. So kann insbesondere ein Drucksensor vorgesehen sein, der den Druckabfall an dem Ventil, den Absolutdruck der Gehirnflüssigkeit oder den Differenzdruck zwischen dem ersten und dem zweiten freien Ende des Katheters mißt. Hierbei werden vorzugsweise mikromechanische Drucksensoren verwendet, die auf einem Siliziumchip integriert sind und somit in besonders kleiner Baugröße herstellbar sind.

Besonders vorteilhaft ist es, zusätzlich einen Flußsensor an der Meßeinheit vorzusehen, der die durch den Katheter strömende Flußmenge pro Zeiteinheit mißt. Zwar ist die Messung des Flusses für die Überwachung des Implantats nicht zwingend erforderlich, da letztlich der Druck der Gehirnflüssigkeit die entscheidende Überwachungsgröße ist. Allerdings hat man durch die Flußmessung eine zusätzliche Kontrollmöglichkeit der Funktion des Ventils.

Vorzugsweise besteht der Flußsensor aus einem definierten Strömungswiderstand und einem Drucksensor, wobei der Drucksensor den Druckabfall an dem Strömungswiderstand mißt. Der durch den Drucksensor gemessene Differenzdruck ist dann eine Funktion des Flusses, so daß der Durchfluß durch den Katheter auf diese Weise bestimmbar ist.

Eine andere bevorzugte Ausführungsform eines Durchflußsensors kann darin bestehen, daß der Durchflußsensor aus einem Kompensationsregler besteht, der den Differenzdruck zwischen dem Zufluß und dem Abfluß eines Meßrohres über eine in das Meßrohr eingebrachte Zahnradpumpe ausregelt. Dieses Kompensationsprinzip ist auch noch zur Messung kleinster Durchflußmengen geeignet.

Eine weitere bevorzugte Ausführungsform eines Flußsensors basiert auf dem anemometrischen Funktionsprinzip. Hierbei wird entweder die Leistung bestimmt, die benötigt wird, um die vorbeiströmende Flüssigkeit definiert zu erwärmen oder es wird die Temperaturerhöhung der vorbeiströmenden Flüssigkeit bei definierter Heizleistung ermittelt. Bei einer Realisierung in Siliziumtechnologie kann hiermit ein sehr energiesparender Sensor realisiert werden.

Nach einer weiteren bevorzugten Ausführungsform kann der Durchflußsensor durch einen in der Strömung befindlichen Rotor realisiert werden, dessen Umdrehungsgeschwindigkeit eine Funktion des Durchflusses ist. Die Auswertung der Rotorgeschwindigkeit erfolgt üblicherweise durch eine optische Auswertevorrichtung oder mittels magnetisch-induktiver oder magnetisch-resistiver Prinzipien.

Nach einer weiteren bevorzugten Ausführungsform ist vorgesehen, daß an die Meßeinheit ein Lagesensor angeschlossen ist, der die Lage des Körpers relativ zu dem Erdbeschleunigungsvektor mißt. Als Maß für die Lage des Patienten kann zweckmäßigerweise der Differenzdruck zwischen dem ersten und dem zweiten freien Ende des Katheters oder eine Differenzdruckmessung an zwei Stellen im Katheter dienen. Der Lagesensor kann aber auch durch eine Geschwindigkeits- oder Beschleunigungsmessung realisiert werden, wobei die Körperlage bezüglich des Erdschwerefeldes rechnerisch ermittelt wird. Dabei wird beispielsweise ein Pendel zur Bestimmung der Körperlage verwendet, dessen Position elektrisch erfaßt wird. Eine weitere Möglichkeit besteht in der Messung von Rotations- oder Linear- Beschleunigung oder Geschwindigkeit und einer rechnerischen oder elektrischen Nachverarbeitung zur Ermittlung der Körperlage.

Nach einer weiteren bevorzugten Ausführungsform ist vorgesehen, daß die Telemetrieeinheit eine Recheneinheit aufweist, die die von der Meßeinheit erfaßten Meßwerte aufgrund vorgegebener Sollwerte auswertet. Beispielsweise kann der gemessene Druck oder Durchfluß durch das Implantat mit einem vorgegebenen typischen Druck- oder Durchflußverhalten verglichen werden, wobei nur bei einer bestimmten Abweichung eine Abspeicherung der jeweiligen Daten erfolgt. Auf diese Weise kann bei großen anfallenden Datenmengen seitens der Meßeinheit eine Vorauswahl der relevanten Daten getroffen werden, um möglichst über einen langen Zeitraum Daten in den Meßwertspeicher abspeichern zu können. Besonders vorteilhaft ist es, wenn in der Recheneinheit Testroutinen implementiert sind, die die Funktionsfähigkeit des Implantats überprüfen.

Nach einer weiteren bevorzugten Ausführungsform ist vorgesehen, daß in die Meßeinheit ein Meßwertspeicher integriert ist, in dem vergangene Meßwerte abspeicherbar sind. Auf diese Weise können die Daten der an die Meßeinheit angeschlossenen Sensoren auch unter Alltagsbedingungen erfaßt werden und bei einer Routineuntersuchung des behandelnden Arztes über die Telemetrieeinheit zu der Kontrolleinheit übertragen werden. In der Kontrolleinheit kann dann eine Auswertung der jeweiligen Meßdaten stattfinden, woraufhin dann weitere Therapieschritte festgelegt werden können. Auf diese Weise ist bereits eine Überwachung herkömmlicher passiver Sicherheitsventile mit Rückflußsperre möglich.

Um den Durchfluß durch den Katheter seitens der Telemetrieeinheit zu steuern, kommen grundsätzlich zwei Arten von Ventilen in Frage: Ventile mit veränderbarem Strömungswiderstand und Überströmventile mit veränderlicher Ventilcharakteristik. Die Veränderung des Strömungswiderstandes oder der Ventilcharakteristik kann entweder kontinuierlich oder in diskreten Schritten erfolgen.

Eine Ausführungsform des Ventils besteht demnach aus einem Proportionalventil mit einem in einem Ventilsitz bewegbaren Stempel, wobei der Stempel durch einen seitens der Stelleinheit steuerbaren Antrieb antreibbar ist. Der Strömungswiderstand kann aber auch durch eine in einem Konus bewegbare Kugel oder durch eine Quetschung eines Katheterschlauches erfolgen. Das Proportionalventil ist demnach aktiv, da es für die Arbeit gegen den Flüssigkeitsdruck Energie benötigt.

Eine andere Ausfürhungsform des Ventils besteht aus einem Schaltventil mit nur einen geschlossenen Zustand (d.h. einen unendlich großen Strömungswiderstand) und einen geöffneten Zustand auf, zwischen denen hin- und hergeschaltet wird. Das Taktverhältnis zwischen beiden Zuständen bestimmt den mittleren Strömungswiderstand und damit den Durchfluß. Vorzugsweise weist das Ventil eine Schaltmembran auf, die ohne äußere Krafteinwirkung zwei stabile Zustände einnehmen kann, wobei im einen Zustand der Durchfluß des Katheters gesperrt und im anderen Zustand der Durchfluß des Katheters freigegeben ist und wobei aufgrund magnetischer Krafteinwirkung die Schaltmembran zwischen beiden Zuständen hin- und herschaltbar ist. Die magnetische Krafteinwirkung kann dadurch realisiert sein, daß auf der Schaltmembran eine Spule aufgebracht ist, mit der durch Beaufschlagung mit elektrischem Strom ein Magnetfeld erzeugbar ist und daß an den Seitenwänden des Ventilgehäuses mindestens ein Permagnentmagnet derart befestigt ist, daß die Schaltmembran durch ein entsprechend aufgeschaltetes Magnetfeld der stromdurchflossenen Spule umschaltbar ist. Insbesondere kann die Umschaltung zwischen diesen Positionen durch eine kurzzeitig stromdurchflossene Spule realisiert werden, deren Feld das des haltenden Permanentmagneten abschwächt. Auch das Schaltventil ist demnach ein aktives Ventil, da gegen den Flüssigkeitsdruck beim Schalten Arbeit aufgewendet werden muß.

Eine weitere Ausführungsform des Ventils ist ein Überströmventil mit veränderlicher Ventilcharakteristik. Dieses Ventil ist ein passives Ventil mit aktiver Verstellung des Öffnungsdrucks. Das Ventil selbst ist energielos, die Stellenergie kommt aus einem passiven Energiespeicher, beispielsweise einer vorgespannten Feder oder Membran. Die Verstellung kann entweder kontinuierlich oder in diskreten Stufen erfolgen.

Als Antrieb für die verschiedenen Ventilausführungen eignet sich z.B. ein sogenannter Inchworm-Motor, der aus zwei piezoelektrischen Halteelementen besteht, die im Gegentakt einen piezoelektrischen Stab in Abhängigkeit von dessen Ausdehnungsrichtung halten oder freigeben. Die Ausdehnung des piezoelektrischen Stabs oder die Haltefunktion der Halteelemente werden dabei durch Anlegen einer Spannung an die jeweiligen Piezoelemente erreicht.

Es kann auch ein Stapel von Piezoelementen zum direkten Antrieb der Ventileinheit verwendet werden, über die die nötige Längenänderung direkt erreicht werden kann.

Eine andere Möglichkeit besteht darin, daß der Antrieb ein ausdehnbarer Balg ist, wobei das Volumen aufgrund eines Phasenübergangs des in dem Balg eingeschlossenen Mediums veränderbar ist. Der Phasenübergang kann beispielsweise durch Aufheizung hervorgerufen werden, so daß der Ventilsitz geschlossen oder geöffnet werden kann. Die Volumenänderung des im Balg eingeschlossenen Mediums kann auch durch elektrochemische (Elektrolyse) oder elektrophysikalische (z.B. wie bei elektrisch sensitiven Gelen) Effekte hervorgerufen werden.

Eine weitere Möglichkeit des Antriebs besteht darin, daß der Antrieb ein miniaturisierter Elektromotor mit einem selbsthemmenden Getriebe ist, wobei das selbsthemmende Getriebe die Rotationsbewegung der Motorachse in eine Linearbewegung des Stempels umwandelt. Durch das selbsthemmende Getriebe braucht keine zusätzliche Energie für die Haltekraft des Stempels aufgebracht werden, so daß ein derartiger Antrieb besonders energiesparend ist.

Schließlich ist als Antrieb auch eine Tauchspule in einem permanenten Magnetfeld denkbar. Eine solche Anordnung ist, wie bereits oben beim Schaltventil beschrieben, für die Realisierung eines Ventils mit diskreten Stellpositionen besonders günstig.

Besteht somit eine Verstellmöglichkeit des Ventils, eröffnen sich für den behandelnden Arzt völlig neue Therapiemöglichkeiten, da dieser nach Auswerten der von der Telemetrieeinheit übertragenen Meßdaten den Durchflußwiderstand des Ventils neu verstellen kann. Gegebenenfalls kann dies auch automatisch durch die Kontrolleinheit erfolgen.

Zusätzlich oder alternativ zu der Möglichkeit der Verstellung des Ventils durch die Kontrolleinheit kann auch ein Regler vorgesehen sein, der in Abhängigkeit von mindestens einer gemessenen Zustandsgröße des Ventils und einem vorgegebenen Sollwert eine Stellgröße für das Stellventil derart bestimmt, daß das Abflußverhalten der Gehirnflüssigkeit dem Sollwert entspricht. Ein derartiger Regler kann in der Kontrolleinheit oder aber auch in der Telemetrieeinheit integriert sein.

Eine solche Zustandsgröße könnte beispielsweise die Lage des Körpers relativ zu dem Erdbeschleunigungsvektor sein. Auf Grund der Sensorinformation könnte dann der Sollwert eines Regelkreises verstellt werden oder ein direkter Stelleingriff im Sinne einer Steuerung am Aktor erfolgen. Im regelungstechnischen Sinne wird dadurch eine Störgrößenaufschaltung realisiert.

Falls der Regler in der Kontrolleinheit integriert ist, kann das Ventil während eines Routinebesuchs beim behandelnden Arzt neu eingestellt werden. Ist aber der Regler in der Telemetrieeinheit integriert, kann die Telemetrieeinheit eine Verstellung des Ventils aufgrund der aufgenommenen Meßdaten kontinuierlich und selbständig vornehmen. In diesem Fall kann die notwendige Datenübertragung zur Kontrolleinheit auf ein Minimum beschränkt werden, da der in der Telemetrieeinheit integrierte Regler von sich aus eine optimale Funktion des Ventils gewährleistet. Dennoch wird es zweckmäßig sein, bei Routineuntersuchungen die unter Alltagsbedingungen aufgenommenen Meßdaten des Implantats zu dem Kontrollsystem zu übertragen und außerdem seitens des Kontrollsystems gegebenenfalls einen neuen Sollwert für den Regler vorzugeben.

Eine einfache Realisierungsform des Reglers könnte beispielsweise darin bestehen, daß der Regler ein PI-Regler ist, der die Regeldifferenz zwischen einem vorgegebenen Druck-Sollwert und einem gemessenen Druck der Gehirnflüssigkeit in eine Stellgröße für den Antrieb des Stellventils umwandelt. Als Regelgröße kommt aber auch der Liquorfluß durch den Shunt in Betracht, wobei man in diesem Fall einen gewisse Liquormengenbereich pro Tag vorgeben würde unter Beachtung geeigneter Grenzwerte für den Hirndruck. Selbstverständlich sind aber auch alle anderen Arten von geeigneten Regelgrößen und Reglertypen denkbar.

Nach einer weiteren bevorzugten Ausführungsform ist vorgesehen, daß die Telemetrieeinheit und die Kontrolleinheit einen Schwingkreis mit jeweils einer Induktivität aufweisen, wobei bei einer Übertragung von Meßwerten der Schwingkreis seitens der Telemetrieeinheit und bei einer Übertragung von Stellwerten der Schwingkreis seitens der Kontrolleinheit verstimmbar ist und daß die Datenübertragung berührungslos durch induktive Kopplung der Induktivitäten erfolgt. Auf diese Weise ist eine bidirektionale Datenübertragung ohne großen Energiebedarf seitens der Telemetrieeinheit möglich. Die Information kann entweder in analoger oder digitaler Form kodiert werden. Statt den Informationsgehalt der übertragenen Frequenz auszuwerten, kann man auch deren Energiegehalt zur Aufladung eines Akkumulators oder eines Kondensators nutzen, die ihrerseits zur Energieversorgung der Telemetrieeinheit dienen. Auf diese Weise kann die Größe und das Gewicht der Telemetrieeinheit weiter reduziert werden, da eine regelmäßig Aufladung eines in der Telemetrieeinheit vorgesehenen Energiespeichers bei den ohnehin notwendigen Kontrolluntersuchungen möglich ist.

Die Kontrolleinheit ist zweckmäßigerweise an ein Computersystem angeschlossen, das die übertragenen Meßwerte auswertet und das die zu übertragenden Stellwerte berechnet. Beispielsweise kann die Kontrolleinheit aus einem handlichen Tastkopf bestehen, der zur Datenübertragung in die Nähe der implantierten Telemetrieeinheit geführt wird. In dem Tastkopf sind dabei nur die zur Datenübertragung notwendigen Elemente enthalten, während in dem Computersystem die eigentliche Auswertung stattfindet.

Weitere Eigenschaften und Vorteile der Erfindung werden anhand eines in der Zeichnung dargestellten Ausführungsbeispiels näher erläutert. In dieser zeigt;
- Fig. 1:: eine schematische Darstellung der an die erfindungsgemäße Telemetrieeinheit angeschlossenen Komponenten,
- Fig. 2:: ein Blockschaltbild der erfindungsgemäßen Telemetrieeinheit,
- Fig. 3:: ein elektrisches Prinzipschaltbild für die Datenübertragung von der Telemetrieeinheit zur Kontrolleinheit,
- Fig. 4:: ein elektrisches Prinzipschaltbild für die Datenübertragung von der Kontrolleinheit zur Telemetrieeinheit,
- Fig. 5:: eine schematische Darstellung des gesamten Implantats,
- Fig. 6:: eine schematische Darstellung eines Aktors und eines Stellventils,
- Fig. 7:: eine schematische Darstellung eines Proportionalventils,
- Fig. 8:: eine schematische Darstellung eines Schaltventils,
- Fig. 9:: eine schematische Darstellung eines Durchflußsensors,
- Fig. 10:: eine schematische Darstellung eines Durchflußsensors mit einem Kompensationsregler,
- Fig. 11:: eine schematische Darstellung eines Durchflußsensors nach dem Anemometer-Prinzip,
- Fig. 12:: eine schematische Darstellung der Durchflußmessung mit einem Rotor,
- Fig. 13:: eine schematische Darstellung eines Aktors und eines proportionalen Ventils, bei dem der Querschnitt eines Schlauches verändert wird,
- Fig. 14:: eine schematische Darstellung der Kombination eines Aktors und eines verstellbaren Feder/Kugelventils,
- Fig. 15:: eine schematische Darstellung eines diskreten Stellventils,
- Fig. 16:: eine Prinzipskizze eines Balg-Antriebes,
- Fig. 17:: eine schematische Darstellung eines möglichen Neigungssensors und
- Fig. 18:: eine schematische Darstellung eines weiteren möglichen Neigungssensors.

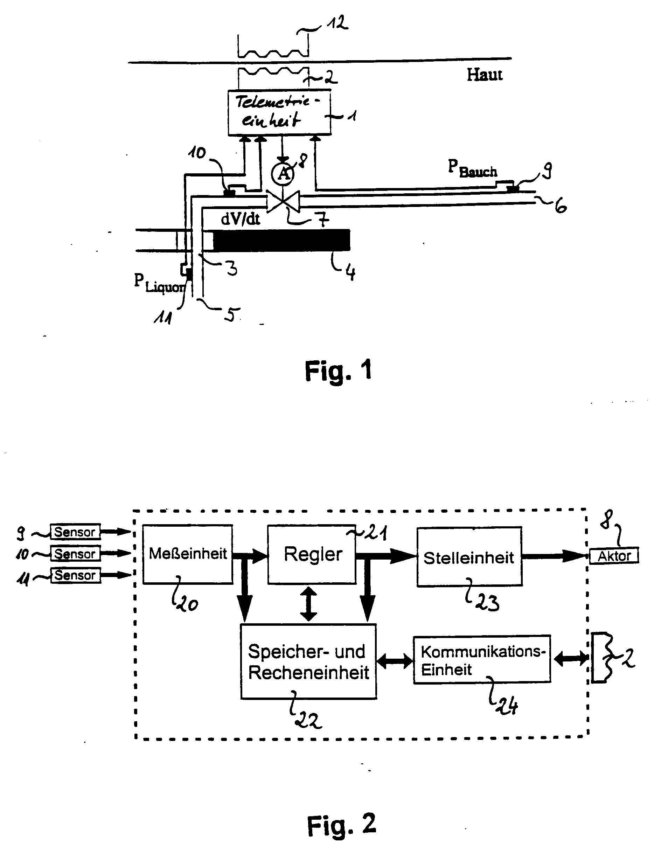

Fig. 1 zeigt eine schematische Darstellung der an die erfindungsgemäße Telemetrieeinheit angeschlossenen Komponenten. Die Telemetrieeinheit 1 ist an einer geeigneten Stelle des Körpers unter der Haut des Patienten implantiert, wobei ein Tastkopf 2 zu der Oberfläche der Haut ausgerichtet ist. Ein Katheter 3 ist durch die Schädeldecke 4 mit seinem ersten freien Ende 5 in das Innere des Gehirns geführt und ist unter der Haut des Patienten derart verlegt, daß das zweite freie Ende 6 in dem Bauchraum des Patienten endet. Auf dem Verlauf des Katheters 3 ist ein Ventil 7 eingebracht, dessen Durchflußwiderstand mit einem Antrieb 8 veränderbar ist. Das Ventil 7 ist vorzugsweise in der Nähe der Telemetrieeinheit implantiert oder bildet mit dieser eine integrierte Einheit. An die Telemetrieeinheit 1 sind drei Sensoren 9, 10, 11 angeschlossen, die verschiedene Zustände der in dem Katheter 3 fließenden Gehirnflüssigkeit erfassen. Mit den Sensoren 9 und 11 ist der Druck im Bauchraum bzw. in dem Inneren des Gehirns meßbar, wohingegen mit dem Sensor 10 die Durchflußgeschwindigkeit in dem Katheter 3 meßbar ist. Umgekehrt ist auch der Antrieb 8 mit der Telemetrieeinheit elektrisch verbunden. In der Nähe des Tastkopfes 2 kann im Bedarfsfall von außen auf die Haut des Patienten ein zweiter Tastkopf 12 aufgesetzt werden, der mit einer nicht näher dargestellten Kontrolleinheit verbunden ist. Die Kontrolleinheit kann dabei beispielsweise aus einem Computersystem bestehen.

Fig. 2 zeigt ein Blockschaltbild der erfindungsgemäßen Telemetrieeinheit. Die Telemetrieeinheit besteht aus einer Meßeinheit 20, einem Regler 21, einer Speicher- und Recheneinheit 22, einer Stelleinheit 23 und einer Kommunikationseinheit 24. An die Meßeinheit 20 sind gemäß Fig. 1 die Sensoren 9, 10, 11 angeschlossen. In der Meßeinheit 20 findet dabei eine Aufbereitung und eine AD-Wandlung der Sensorsignale statt. Die digitalen Meßwerte der Sensoren werden sodann sowohl an den Regler 21 als auch an die Speicher- und Recheneinheit 22 weitergeleitet. Der Regler 21 setzt die Meßwerte der Meßeinheit 20 aufgrund des entsprechenden Regelalgorithmus und in Abhängigkeit von dem vorgegebenen Sollwert in einen Stellwert um, der der Stelleinheit 23 zugeführt wird. Die Stelleinheit 23 wandelt den jeweiligen von dem Regler 21 gelieferten Stellwert mittels eines DA-Wandlers in einen analogen Wert um und bereitet diesen entsprechend auf, so daß der analoge Stellwert an den Aktor 8 weitergeleitet werden kann. Aufgrund des Aktors 8 findet gemäß Fig. 1 eine Veränderung des Durchflußwiderstandes des Ventils 7 statt, so daß sich wiederum die Meßgrößen der Sensoren 9, 10, 11 verändern und somit der Regelkreis geschlossen ist. Die Speicher- und Recheneinheit 22 erfaßt zusätzlich die digitalen Meßwerte und die digitalen Stellwerte und speichert diese in regelmäßigen Abständen ab. Zusätzlich führt eine interne Recheneinheit eine Überwachung der erfaßten Werte durch und verändert gegebenenfalls die Parameter des Regelalgorithmus. Eine derartige Adaption der Regelparameter kann auch auf die Fälle beschränkt werden, in denen eine Datenübertragung mit der externen Kontrolleinheit stattfindet. Für diesen Fall kann die Recheneinheit also in die Kontrolleinheit ausgelagert werden.

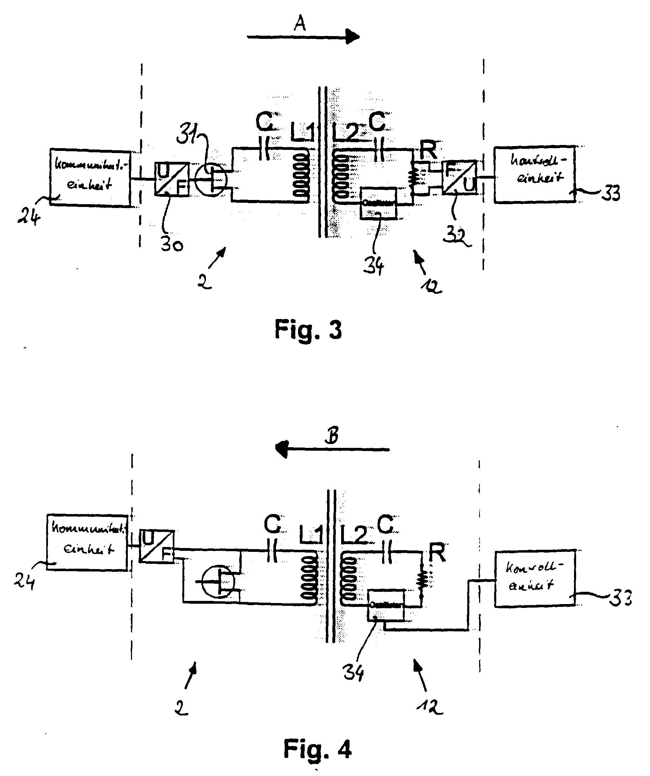

Um eine Datenübertragung zwischen der Telemetrieeinheit und einer externen Kontrolleinheit durchzuführen, wird gemäß Fig. 1 der Tastkopf 12 der Kontrolleinheit in die Nähe des Tastkopfes 2 der Telemetrieeinheit gesetzt. Um die in der Speicher- und Recheneinheit 22 gespeicherten Werte zu der Kontrolleinheit zu übertragen, werden die Werte von der Kommunikationseinheit 24 aus der Speicher- und Recheneinheit 22 ausgelesen, für eine Datenübertragung und dem Tastkopf 2 zugeführt. Eine nähere Beschreibung der Datenübertragung findet sich dabei in Fig. 3. In umgekehrter Weise wird auch eine Datenübertragung zwischen der Kontrolleinheit und der Telemetrieeinheit hergestellt, eine entsprechende Beschreibung findet sich in Fig. 4.

Fig. 3 zeigt ein elektrisches Prinzipschaltbild für die Datenübertragung von der Telemetrieeinheit zu der Kontrolleinheit. Die Übertragungsrichtung ist durch den Pfeil A angedeutet. Die in der Kommunikationseinheit 24 gewandelten Werte werden dem Tastkopf 2 und dort einem Spannungs-Frequenzwandler 30 zugeführt, der einen Schalttransistor 31 taktet, wodurch die Resonanzfrequenz des durch C und L1 gebildeten Schwingkreises entsprechend der Taktfrequenz verändert wird. In dem Tastkopf 12 wird hierdurch die Resonanzfrequenz des durch C und L2 gebildeten Schwingkreises verstimmt, wobei die Sendeenergie durch den in dem Tastkopf 12 befindlichen Oszillator 34 geliefert wird. Über den Widerstand R wird die derart modulierte Resonanzfrequenz über den Frequenz-Spannungswandler in Form einer analogen Spannung der Kontrolleinheit 33 zugeführt.

Fig. 4 zeigt ein elektrisches Prinzipschaltbild für die Datenübertragung von der Kontrolleinheit zu der Telemetrieeinheit. Die Übertragungsrichtung ist durch den Pfeil B angedeutet. In diesem Fall wird die Frequenz des Oszillators 34 entsprechend den zu übertragenden Werten der Kontrolleinheit 33 verändert. Die Datenübertragung erfolgt ansonsten analog gemäß Fig. 3.

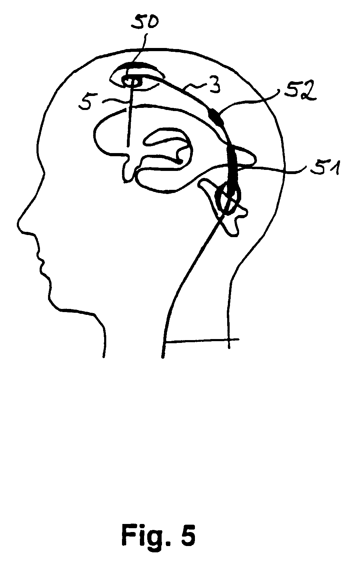

Fig. 5 zeigt eine schematische Darstellung des gesamten Implantats. Auf der oberen Seite des Schädels ist in die Schädeldecke ein Loch 50 eingebracht, in die das erste freie Ende 5 des Katheters 3 eingeführt ist. Der Katheter 3 ist unterhalb der Kopfhaut verlegt und endet mit seinem zweiten freien Ende in dem Bauchraum. Die Telemetrieeinheit und das Ventil bilden eine integrierte Einheit 51, die wie in Fig. 5 abgebildet hinter dem Ohr des Patienten oder vorzugsweise auch unter dem Schlüsselbein implantiert werden kann. Vor der integrierten Einheit 51 ist eine Erweiterung 52 in dem Silikonschlauch vorgesehen, die zur Beseitigung eventueller Verstopfungen oder Verklebungen pumpenartig gedrückt werden kann.

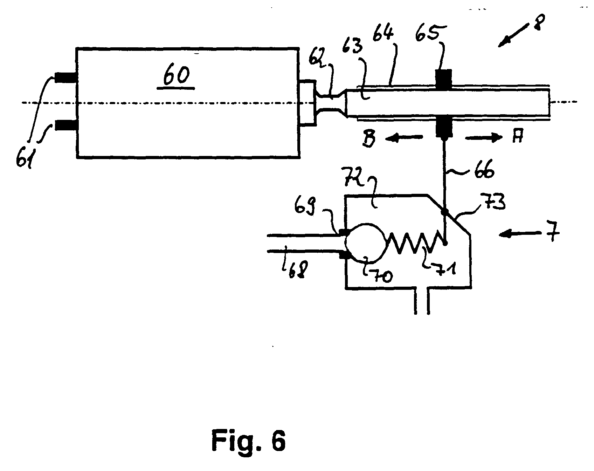

Fig. 6 zeigt eine schematische Darstellung eines Aktors 8 und eines passiven Ventils 7. Der Aktor 8 besteht aus einem miniaturisierten Elektromotor 60, der über die Anschlüsse 61 an- und ausschaltbar ist. Die Antriebswelle des Elektromotors 60 ist mit einer Wellenkupplung 62 mit einer Spindelwelle 63 verbunden. Die Spindelwelle 63 weist ein Gewinde 64 auf, auf dem eine Spindelmutter 65 läuft. Die Spindelmutter 65 ist mit einem Hebelarm 66 gelenkig verbunden, der in die Ventilkammer 72 eines Ventils 7 hineinragt und an dem Gehäuse 73 drehbar gelagert ist.

Das Ventil besitzt einen Zufluß 68, der in einen Konus 69 mündet. Der Konus 69 ist durch eine Kugel 70 verschließbar, wobei die Anpreßkraft durch die Feder 71 aufgebracht wird. Die Feder 71 ist mit dem in die Ventilkammer 72 hineinragenden Hebelarm 66 verbunden, so daß durch Verstellung des Hebelarms die Anpreßkraft auf die Kugel 70 veränderbar ist.

Zur Verstellung der Ventilcharakteristik wird eine Spannung an die Anschlüsse 61 angelegt. In Abhängigkeit von der Drehrichtung der Spindelwelle 63 kann dabei der Öffungsdruck des Ventils 7 vergrößert oder verringert werden. Bewegt sich demnach die Spindelmutter 65 in die mit A gekennzeichnete Richtung, so vergößert sich der Öffnungsdruck, bewegt sich dagegen die Spindelmutter 65 in die mit B gekennzeichnete Richtung, so verringert sich der Öffnungsdruck des Ventils 7.

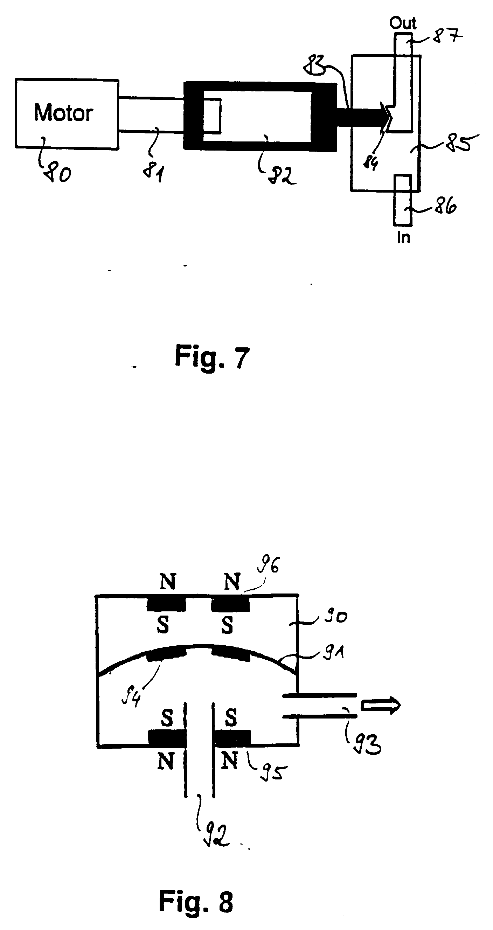

Fig. 7 zeigt eine schematische Darstellung eines Proportionalventils. Das Proportionalventil besteht aus einem miniaturisierten Gleichstrommotor 80, dessen Welle eine Gewindespindel 81 antreibt. Auf der Gewindespindel 81 läuft ein Gewindering 82 mit einem Stempel 83, dessen Ende in einen Ventilsitz 84 hineinragt. Der Ventilsitz 84 ist umgeben von einem Ventilgehäuse 85 mit einem Zufluß 86 und einem Abluß 87, so daß der Durchflußwiderstand zwischen dem Zufluß 86 und dem Abfluß 87 durch Verstellung des Ventilsitzes 84 veränderbar ist.

Fig. 8 zeigt eine schematische Darstellung eines Schaltventils. Das Schaltventil besteht aus einem Ventilgehäuse 90, in dessen Inneren eine Membran 91 zwischen zwei Zuständen hin- und herpendelt. Im ersten Zustand ist durch die Membran 91 der Zufluß 92 verschlossen, während im zweiten Zustand der Zufluß 92 geöffnet ist, so daß Flüssigkeit durch das Ventilgehäuse 90 zum Ausfluß 93 strömen kann. Auf der Membran 91 befindet sich eine umpolbare Spule 94. Dazu zentriert sind an den angrenzenden Seitenwänden des Ventilgehäuse jeweils gegensinnig gepolte Permanentmagneten befestigt. Hierdurch kann durch Anlegen einer Spannung an die umpolbare Spule der Schaltzustand der Membran 91 verändert werden.

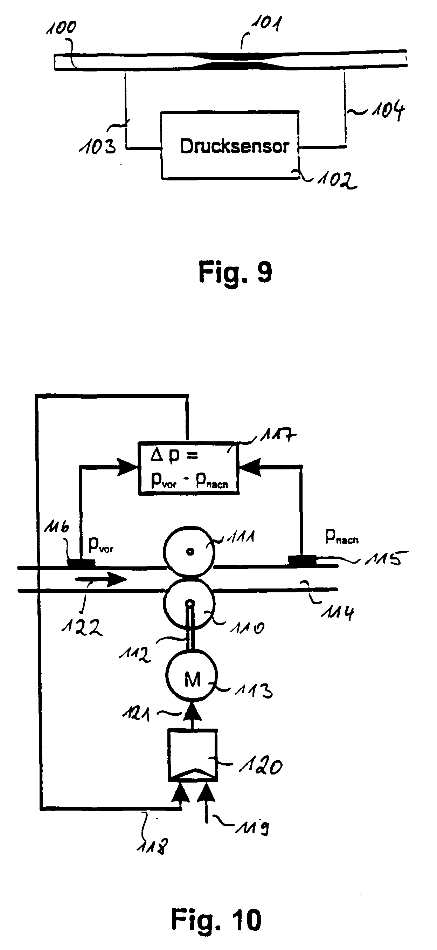

Fig. 9 zeigt eine schematische Darstellung eines Durchflußsensors. Der Durchflußsensor besteht aus einem Meßrohr 100, in das ein Durchflußwiderstand 101 eingebracht ist. Vor und hinter dem Durchflußwiderstand wird Flüssigkeit durch die Leitungen 103 und 104 zu einem Drucksensor 102 geführt, so daß der Druckabfall über den Strömungswiderstand 101 gemessen wird. Der von dem Drucksensor 102 gemessene Differenzdruck ist dabei eine Funktion des Flusses. Als Drucksensoren kommen handelsübliche Drucksensoren in Frage, wobei sich aufgrund der Miniaturisierung insbesondere mikromechanische Drucksensoren eigenen.

Fig. 10 zeigt eine schematische Darstellung eines Durchflußsensors mit einem Kompensationsregler. Der Durchflußsensor weist eine Zahnradpumpe mit Zahnrädern 110 und 111 auf die über ein Getriebe 112 von einem Elektromotor 113 angetrieben werden. Die Zahnräder 110 und 111 sind derart in ein Meßrohr 114 eingebracht, daß diese bei Anlegen eines Stellsignals 121 die in dem Meßrohr 114 befindliche Flüssigkeit in Richtung des Pfeiles 122 beschleunigen.

Der Differenzdruck der in dem Meßrohr fließenden Flüssigkeit wird zwischen den Stellen 115 und 116 durch den Drucksensor 117 gemessen. Das Ausgangssignal 118 des Drucksensors wird zusammen mit einem Sollwert 119 einem Regler 120 zugeführt. Vorzugsweise ist der Sollwert 119 identisch Null, so daß der Regler 120 über die Zahnradpumpe den durch den Drucksensor 117 gemessenen Differenzdruck ausregelt. Die Stellgröße 121 ist in diesem Fall direkt proportional zum Durchfluß durch das Meßrohr.

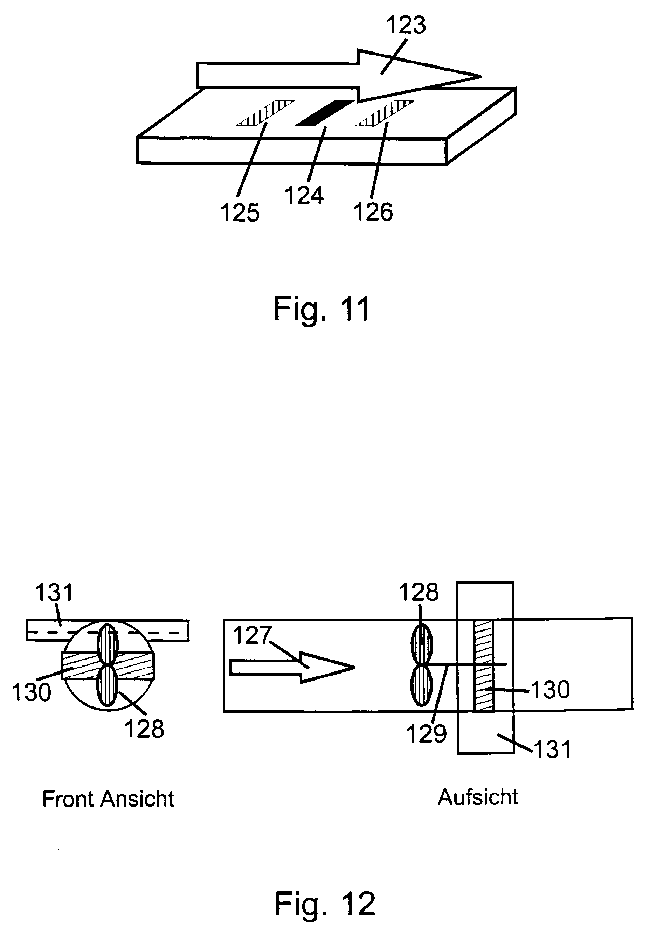

Fig. 11 zeigt die schematische Darstellung eines Durchflußsensors nach dem Anemometer-Prinzip. Im Flüssigkeitsstrom 123 befindet sich ein Heizelement 124, das diesen erwärmt. Zwei Temperatursensoren 125 und 126 messen die Temperatur des vorbeiströmenden Mediums. Bei konstanter Heizleistung kann aus dem Temperaturgradienten bei bekannter Wärmekapazität des Fluids der Durchfluß bestimmt werden. Alternativ kann die Heizleistung so geregelt werden, daß sich immer ein definierter Temperaturgradient einstellt. In diesem Fall ist die Heizleistung ein Maß für den Durchfluß.

Fig. 12 zeigt die schematische Darstellung der Durchflußmessung mit einem Rotor. In den Flüssigkeitsstrom 127 wird ein geeignet geformter Rotor 128 eingebracht, der durch diesen in Rotation versetzt wird. Der Rotor ist über eine Welle 129 in einer Halterung 130 beweglich gelagert. Er unterbricht durch seine Rotationsbewegung eine optische Meßstrecke 131. Die gemessene Unterbrechungsfrequenz ist eine Funktion des Durchflusses.

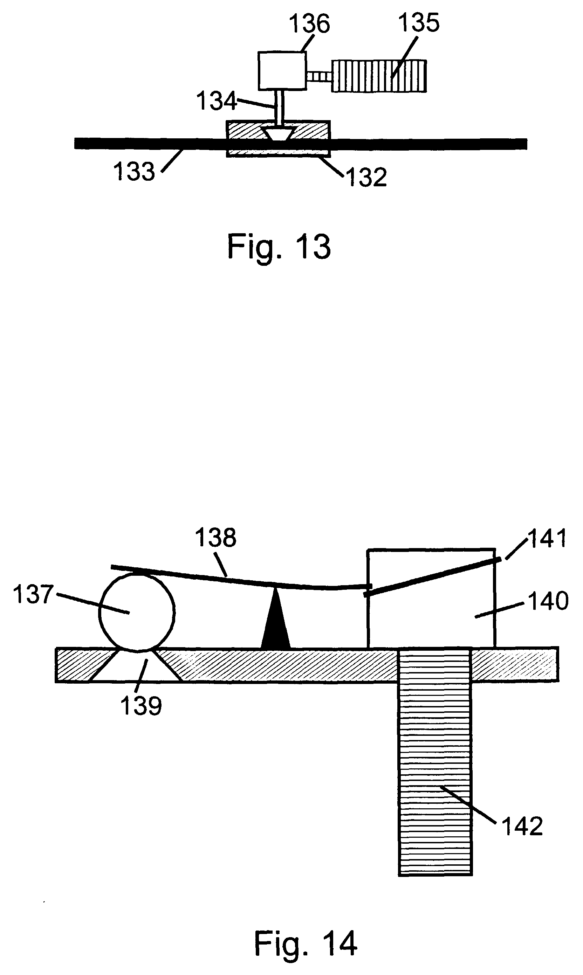

Fig. 13 zeigt eine schematische Darstellung eines Aktors und eines proportionalen Ventils, bei dem der Querschnitt eines Schlauches verändert wird. Ein in eine Fixierung 132 eingelegter Schlauch 133 wird durch einen Stempel 134 in seinem Querschnitt verändert. Die Rotationsbewegung eines Antriebes 135 wird dazu in einem Getriebe 136 in eine Linearbewegung des Stempels umgesetzt.

Fig. 14 zeigt eine schematische Darstellung der Kombination eines Aktors und eines verstellbaren Feder/Kugelventils. Eine Kugel 137 wird durch eine Feder 138 in einen Ventilsitz 139 gedrückt. Die Vorspannung der Feder kann durch einen drehbar gelagerten Zylinder 140 mit einer schrägen, umlaufenden Auswölbung 141, auf der das andere Ende der Feder aufliegt, verändert werden. Der Zylinder wird durch einen elektromotorischen Antrieb 142 bewegt.

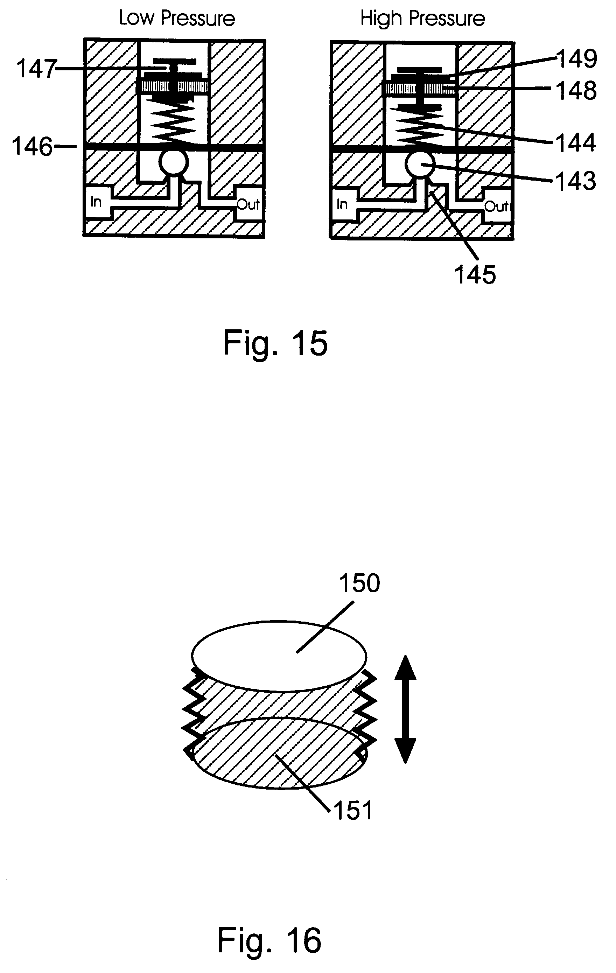

Fig. 15 zeigt die schematische Darstellung eines diskreten Stellventils. Eine Kugel 143 wird durch eine Feder 144 in einen Ventilsitz 145 gedrückt. Eine Membran 146 trennt den Flüssigkeitsraum von den bewegten Komponenten. Die Vorspannung der Feder kann über einen beweglichen Stempel 147 verändert werden. In der "low pressure"-Stellung ist der Weg des Stempels durch einen Anschlag begrenzt. In der "high pressure"- Stellung wird der magnetische Stempel durch einen Permanentmagneten 148 gehalten. Der Wechsel zwischen beiden Schaltstellungen erfolgt durch die Magnetkraft einer stromdurchflossenen Spule 149. Je nach Polung des Stromflusses wird der Stempel abgestoßen oder angezogen.

Fig. 16 zeigt eine Prinzipskizze eines Balg-Antriebes. In einem Faltenbalg 150 befindet sich ein Medium 151, das unter der Einwirkung elektrischer Energie sein Volumen verändert. Da der Balg nur in einer Richtung elastisch ist, wird die Volumenänderung in eine Linearbewegung in Längsrichtung des Balges umgesetzt.

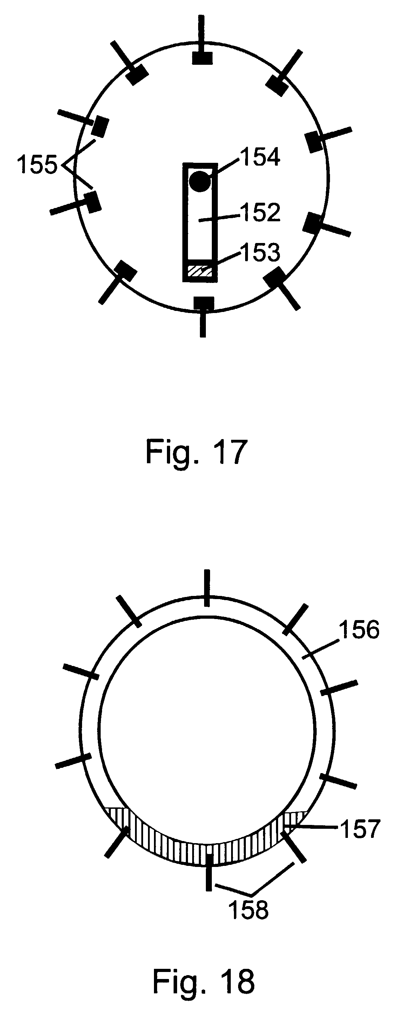

Fig. 17 zeigt die schematische Darstellung eines möglichen Neigungssensors. Ein Pendel 152 mit einem Magneten 153 an der Spitze ist auf einer Welle 154 drehbar gelagert. Auf dem Drehkreis des Pendels sind magnetisch betätigbare Näherungsschalter 155 angebracht. Die Magnetkraft wird so gewählt, daß nur der Schalter ausgelöst wird, der der Pendelspitze am nächsten liegt.

Fig. 18 zeigt die schematische Darstellung eines weiteren möglichen Neigungssensors. In eine Kapillare 156 befindet sich eine elektrisch leitende Flüssigkeit 157. Am Umfang der Kapillare sind elektrische Kontakte 158 angebracht, die je nach Ausrichtung der Vorrichtung durch die elektrisch leitende Flüssigkeit geschlossen werden.

## Patentansprüche

1. Implantat zur kontrollierten Ableitung von Gehirnflüssigkeit, insbesondere zur Therapie des Wasserkopfes,
mit einem unter der Haut verlegten Katheter, dessen erstes freies Ende zu einer Stelle im Gehirn geführt ist, von wo überschüssige Gehirnflüssigkeit ableitbar ist, und dessen zweites freies Ende zu einer Stelle im Körper geführt ist, von wo die überschüssige Gehirnflüssigkeit vom Körper aufnehmbar ist, und
mit einem Ventil, das in dem Katheter auf dessen Verlauf eingebracht ist,
**dadurch gekennzeichnet,**
daß eine Telemetrieeinheit vorgesehen ist, die eine Meßeinheit mit mindestens einem daran angeschlossenen Sensor und eine Stelleinheit mit mindestens einem daran angeschlossenen Aktor aufweist, wobei mindestens ein Zustand des Implantats seitens der Telemetrieeinheit über die Meßeinheit meßbar und über die Stelleinheit verstellbar ist,
und daß zwischen der Telemetrieeinheit und einer außerhalb des Körpers befindlichen Kontrolleinheit eine Datenübertragung und/oder eine Energieübertragung herstellbar ist.

2. Implantat nach Anspruch 1, dadurch gekennzeichnet, daß an die Meßeinheit ein Drucksensor angeschlossen ist, der den Druckabfall an dem Ventil oder den Absolutdruck im Gehirn oder den Differenzdruck zwischen dem ersten und dem zweiten freien Ende des Katheters mißt.

3. Implantat nach einem der Ansprüche 1-2, dadurch gekennzeichnet, daß an die Meßeinheit ein Lagesensor angeschlossen ist, der die Lage des Körpers relativ zu dem Erdbeschleunigungsvektor mißt.

4. Implantat nach einem der Ansprüche 1-3, dadurch gekennzeichnet, daß die Telemetrieeinheit eine Recheneinheit aufweist, die die von der Meßeinheit erfaßten Meßwerte aufgrund vorgegebener Sollwerte auswertet.

5. Implantat nach einem der Ansprüche 1-4, dadurch gekennzeichnet, daß in die Meßeinheit ein Meßwertspeicher integriert ist, in dem vergangene Meßwerte abspeicherbar sind.

6. Implantat nach einem der Ansprüche 1-5, dadurch gekennzeichnet, daß das Ventil ein Schaltventil mit einer zwischen zwei Zuständen hin- und herschaltbaren Membran ist, wobei der Durchflußwiderstand durch das Taktverhältnis an der Membran veränderbar ist.

7. Implantat nach einem der Ansprüche 1-5, dadurch gekennzeichnet, daß der Durchfluß des Ventils durch eine Kugel verschließbar ist, die von einer Feder gegen einen Konus gedrückt wird, wobei die Anpreßkraft der Kugel gegen den Konus durch den Federweg vorgegeben ist, und daß die Federvorspannung durch einen elektrisch ansteuerbaren, selbsthemmenden Linearantrieb verstellbar ist, der im Körper des Patienten implantierbar ist.

8. Implantat nach einem der Ansprüche 1-5, dadurch gekennzeichnet, daß der Durchflußwiderstand des Ventils durch einen in einen Stempel hineinragenden Ventilsitz veränderbar ist, und daß der Stempel durch einen elektrisch ansteuerbaren, selbsthemmenden Linearantrieb verstellbar ist, der im Körper des Patienten implantierbar ist.

9. Implantat nach einem der Ansprüche 1-8, dadurch gekennzeichnet, daß ein Regler vorgesehen ist, der in Abhängigkeit von mindestens einer gemessenen Zustandsgröße des Ventils und einem vorgegebenen Sollwert eine Stellgröße für das Stellventil derart bestimmt, daß das Abflußverhalten der Gehirnflüssigkeit dem Sollwert entspricht.

10. Implantat nach einem der Ansprüche 1-9, dadurch gekennzeichnet, daß auf Grund der durch den Lagesensor ermittelten Position des Patienten bezüglich des Erdschwerefeldes entweder der Sollwert eines Regelkreises verstellt wird oder ein direkter Stelleingriff am Aktor im Sinne einer Steuerung oder Störgrößenaufschaltung erfolgt.
